# EUROPÄISCHE PATENTSCHRIFT

(11) **EP 2 452 759 B1**
(45) Veröffentlichungstag und Bekanntmachung des Hinweises auf die Patenterteilung: **30.04.2014**
(21) Anmeldenummer: 11008983.6
(22) Anmeldetag: 11.11.2011
(51) Int. Cl.: B08B 3/02, A47L 15/23

(54) **Wascharm für eine Reinigungsmaschine zum Reinigen von medizinischen, pharmazeutischen und/oder Labor-Utensilien**
Washing arm for a cleaning machine for cleaning medical, pharmaceutical and/or laboratory utensils.
Bras de lavage pour une machine de nettoyage destinée à nettoyer des ustensiles médicaux, pharmaceutiques et/ou de laboratoire

(30) Priorität: 12.11.2010 DE 102010051218
(43) Veröffentlichungstag der Anmeldung: 16.05.2012
(73) Patentinhaber: Belimed AG, 6300 Zug (CH)
(72) Erfinder: Althammer, Jürgen, 84559 Kraiburg a. Inn (DE); Fromberger, Helmut, 84478 Waldkraiburg (DE)
(74) Vertreter: Feldkamp, Rainer

(56) Entgegenhaltungen:
- EP-A2- 1 238 622
- DE-A1- 1 628 755
- GB-A- 2 314 009
- US-A- 3 960 328
- US-A- 5 964 232

## Beschreibung

Die Erfindung bezieht sich auf einen Wascharm für eine Reinigungsmaschine zum Reinigen von medizinischen, pharmazeutischen und/oder Labor-Utensilien, wobei der Wascharm durch eine Drehkupplung zur Dreh-Lagerung des Wascharms um eine im Wesentlichen vertikale Drehachse und zur Zuführung von Waschmittel und/oder Trocknungsluft in zwei Hälften (3a, 3b) unterteilt ist, die jeweils Austritts-Düsen (31, 61) zur Erzeugung von Waschflüssigkeits-Strahlen (5a, 5b, 6) mit einer vorgegebenen Strahlrichtung aufweisen.

Es sind Reinigungsmaschinen beispielsweise nach Art einer Geschirrspülmaschine oder zum Reinigen von medizinischen, pharmazeutischen und/oder Labor-Utensilien bekannt, die einen Waschraum aufweisen, in den ein Aufnahmewagen zur Aufnahme der zu reinigenden Gegenstände einsetzbar ist, wobei der Begriff "Reinigung" hierbei sowohl das Beaufschlagen der einzelnen Utensilien oder Gegenstände mit einem Strömungsmedium, beispielsweise einer Waschflüssigkeit, einer Sprühflüssigkeit und/oder Trocknungsluft, einschließen soll. Derartige Waschgut-Aufnahmewagen weisen zumindest einen drehbaren Wascharm auf, der um eine vertikale Drehachse drehbar ist, die gleichzeitig eine Drehkupplung zur Zuführung des Strömungsmediums, über Kanäle bildet, die beim Einschieben des Aufnahmewagens in den Waschraum mit einer in dem Waschraum angeordneten Zuführungseinrichtung verbunden werden.

Diese Wascharme weisen jeweils Austritts-Düsen zur Erzeugung von Waschflüssigkeits-Strahlen mit einer vorgegebenen Strahlrichtung auf, die unter einem Winkel von mehr als 0 Grad und weniger als 90 Grad zur Drehachse der Drehkupplung verläuft. Durch entsprechende Gestaltung der Austrittsrichtung der Strömungsmedium-Strahlen aus den Wascharmen wird ein Antrieb der Wascharme um deren Drehachse erreicht.

Aus der DE 16 28 755 A1 ist eine Geschirrspülmaschine bekannt, die Wascharme aufweist, an deren Enden sogenannte Treibdüsen angeordnet sind, die eine Drehbewegung in der Horizontalen um eine Mittelachse hervorrufen. Auf diese Weise wird der Antrieb der Wascharme unabhängig von den aus dem entlang der Längsachse der Wascharme austretenden Waschflüssikeits-Strahlen, so dass sich ein Drehantrieb der Wascharme selbst dann ergibt, wenn die Waschflüssigkeits-Strahlen bei Betrachtung von einem Ende eines Wascharmes aus beispielsweise unter einem Winkel von 0 Grad gegenüber der Drehachse der Drehkupplung ausgestoßen werden.

Der Erfindung liegt die Aufgabe zugrunde, einen Wascharm der eingangs genannten Art zu schaffen, der sich eine wesentliche Verbesserung der Reinigungswirkung ergibt.

Diese Aufgabe wird durch die im Patentanspruch 1 angegebenen Merkmale gelöst.

Vorteilhafte Ausgestaltungen und Weiterbildungen der Erfindung ergeben sich aus den Unteransprüchen.

Dadurch, dass dass die erste Austritts-Düsen in beiden Hälften des Wascharmes zur Erzeugung von ersten Waschflüssigkeits-Strahlen derart ausgerichtet sind, dass die Strahlrichtungen bei Betrachtung von einem Ende des Wascharmes (3) aus in beiden Hälften (3a, 3b) in die gleiche Richtung gerichtet sind, ergibt sich bei einer Drehung des Wascharms eine abwechselnde Beaufschlagung des Waschguts mit schräg in entgegengesetzten ersten und zweiten schräg verlaufenden Strahlrichtungen, so dass auch schwer zu erreichende Bereiche des Waschguts sicher mit dem Strömungsmedium beaufschlagt werden.

Die Wascharme können in einem Waschraum der Reinigungsmaschine und/oder an einem in den Waschraum einsetzbaren Aufnahmewagen für Waschgut drehbar angeordnet sein.

An den jeweiligen freien Enden der Wascharm-Hälften sind horizontal gerichtete zweite Austritts-Düsen derart angeordnet, dass hieraus austretende zweite Waschflüssigkeitsstrahlen bei Betrachtung von einem Ende des Wascharmes aus in beiden Hälften in einander entgegengesetzte, im Wesentlichen horizontale Richtungen gerichtet sind, um den Antrieb des Wascharmes um die Drehachse der Drehkupplung zu bewirken.

Gemäß einer vorteilhaften Ausgestaltung der Erfindung weisen die Wascharme (3) einen im wesentlichen kreisrunden Querschnitt auf, und die ersten Düsen sind durch Austritts-Bohrungen gebildet, die entlang einer Linie parallel zu einer Längsachse des Wascharmes unter einem Radius ausgerichtet sind, der einen Winkel von mehr als 0 Grad und weniger als 90 Grad gegenüber einem Radius parallel zur Drehachse des Wascharms aufweist. Hierdurch ergibt sich eine sehr einfache Herstellung der Wascharme durch Bohren von Standardbauteilen, wie Rohren, mit einem entsprechenden Bohrbild

Die freien Enden der Wascharm-Hälften können durch Endkappen verschlossen sein, wobei in den Endkappen dritte Austritts-Düsen derart angeordnet sein können, dass in Längsrichtung der Wascharme gerichtete dritte Waschflüssigkeitsstrahlen zur Reinigung des Waschraumes erzeugt werden.

An den Endkappen können weiterhin Halter für zumindest einen Magneten derart angeordnet sein, dass der zumindest eine Magnet gegenüber der Längsachse der Wascharm-Hälften achsversetzt angeordnet ist, wobei die Magnete mit ortsfest angeordneten Magnet-Sensoren für eine Überwachung der Drehung des Wascharms zusammenwirken.

Der zumindest eine Wascharm kann weiterhin Teil einer Wascharm-Anordnung sein, die mehrere an einem vertikalen Tragrohr angeordnete Wascharme aufweist, die jeweils mit dem Tragrohr in Strömungsmittelverbindung stehen, wobei die Drehkupplung an einem unteren und/oder oberen Ende des Tragrohres angeordnet ist. Hierbei kann gegebenenfalls nur ein Teil der Wascharm-Hälften mit den zweiten Austritts-Düsen für den Antrieb des Wascharmes um die Drehachse der Drehkupplung ausgerüstet sein.

Weiterhin können, obwohl im Vorstehenden eine vertikale Drehachse der Wascharme beschrieben würde, die gleichen Prinzipien auch auf einen Wascharm mit horizontaler Drehachse, beispielsweise an einer Wand des Waschraums, angewandt werden.

Die Erfindung wird im Folgenden anhand von der Zeichnung dargestellten Ausführungsbeispielen noch näher erläutert.

In den Zeichnungen zeigen:
Figur 1 eine perspektivische schematische Ansicht eines Aufnahmewagens einer Reinigungsmaschine mit einer Ausführungsform des Wascharms in einer ersten Drehstellung zur Erläuterung der Erfindung;
Figur 2 eine der Figur 1 entsprechende Darstellung, bei der der Wascharm eine Drehung um 180 Grad ausgeführt hat;
Figur 3 eine vereinfachte Ansicht einer Ausführungsform des Wascharms in einer ersten Drehstellung;
Figur 4 eine Ansicht der Ausführungsform des Wascharms nach Figur 3 nach einer Drehung um 180 Grad.;

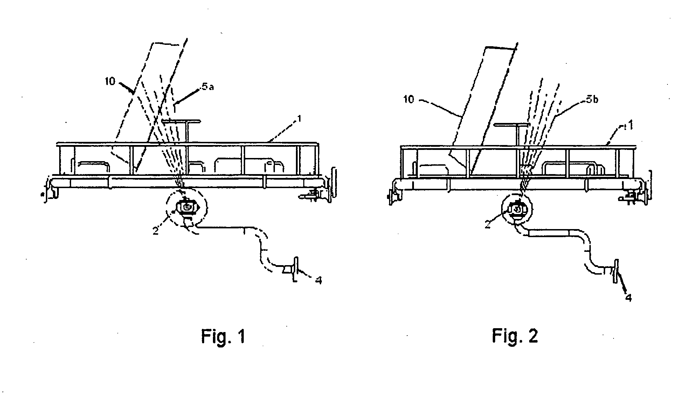

In den Figuren 1 und 2 ist schematisch eine Ansicht eines Waschgut-Aufnahmewagens 1 gezeigt, der in eine nicht gezeigte Aufnahmekammer einer Reinigungs- und/oder Desinfektions- und/oder Trocknungsmaschine (im Folgenden "Reinigungsmaschine") für die Behandlung von medizinischen, pharmazeutischen und/oder Labor-Utensilien einsetzbar oder einschiebbar ist. Dieser Waschgut-Aufnahmewagen 1 weist Auflageflächen für Aufnahmekörbe für zu reinigende Gegenstände oder für die zu reinigenden Gegenstände selbst auf, die nur schematisch in Form einer Schale 10 dargestellt sind.

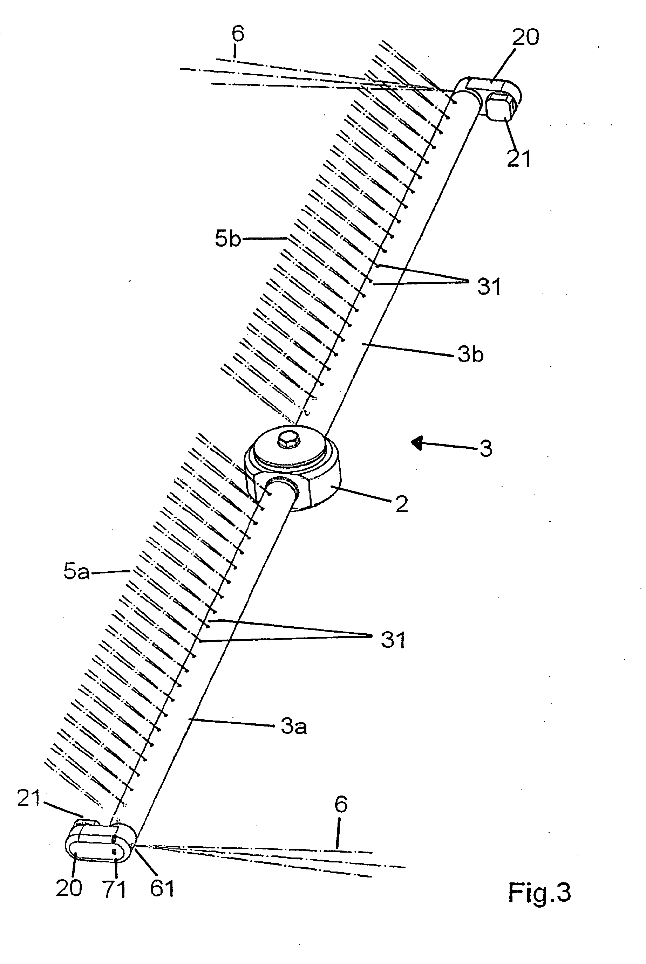

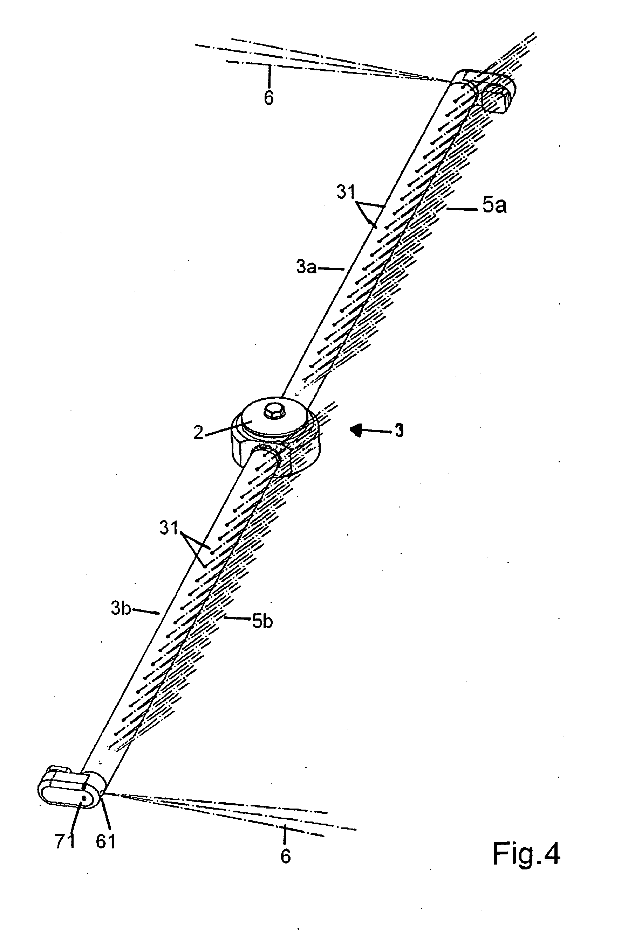

Unter dem Aufnahmewagen 1 ist ein ausführlicher in den Figuren 3 und 4 gezeigter Wascharm 3 mit Hilfe einer Drehkupplung 2 drehbar angeordnet, die mit ebenfalls nur schematisch gezeigten Zuführungseinrichtungen 4 zur Zuführung sowohl von Wasch- oder Spülflüssigkeit als auch von Trocknungsluft an den Wascharm verbunden ist.

Obwohl in den Figuren 1 und 2 nur ein unter einer Ebene oder Etage des Aufnahmewagens angeordneter Wascharm gezeigt ist, können Wascharme selbstverständlich auch unter /und oder über der oder jeder Ebene oder Etage eines Aufnahmewagens mit einer oder mehreren Ebenen angeordnet sein.

Wie dies aus den Figuren 1 bis 4 zu erkennen ist, ist der Wascharm 3 durch die Drehkupplung 2 in zwei Hälften 3a, 3b unterteilt, die jeweils erste Austritts-Düsen 31 zur Erzeugung von Waschflüssigkeits-Strahlen 5a, 5b, 6 mit einer vorgegebenen Strahlrichtung aufweisen, die unter einem Winkel von mehr als 0 Grad und weniger als 90 Grad zur Drehachse der Drehkupplung verläuft, wobei die ersten Austritts-Düsen 31 in beiden Hälften 3a, 3b des Wascharmes 3 zur Erzeugung von ersten Waschflüssigkeits-Strahlen derart ausgerichtet sind, dass die Strahlrichtungen bei Betrachtung von einem Ende des Wascharmes (3) aus (in die Zeichnungsebene nach den Figuren 1 und 2 hinein) in beiden Hälften 3a, 3b in die gleiche Richtung gerichtet sind,

Dies steht im Gegensatz zu herkömmlichen Wacharmen, bei denen die Strahlrichtungen in den beiden Hälften des Wascharms schräg in entgegengesetzte Richtungen gerichtet sind, um den Drehantrieb des Wascharms zu bewirken.

Durch die erfindungsgemäße Ausrichtung der Strahlrichtungen wird sichergestellt, dass zumindest bei einer jeweiligen halben Umdrehung des Wascharms auch für schräg gerichtete Strömungsmittel-Strahlen sonst schwer zugängliche Bereiche des Waschguts sicher mit dem Strömungsmedium beaufschlagt werden.

Dies ist insbesondere aus einem Vergleich der Figuren 1 und 2 zu erkennen, die jeweils die Strahlen.der aus der Zeichnungsebene heraus gerichteten Hälfte des Wascharms 3 zeigen, wobei die Figur 1 der Drehstellung des Wascharms 3 nach Figur 3 entspricht, während die Figur 2 der Drehstellung des Wascharms 3 nach Figur 4 entspricht.

Wenn wie bei herkömmlichen Wascharmen die Strahlen der Wascharm-Hälften in entgegengesetzte Richtungen gerichtet sind, also in Figur 3 die Strahlen der Wascharm-Hälfte 3a nach rechts in der Zeichnung gerichtet wären, könnte das Innere der als Beispiel in den Figuren 1 und 2 dargestellten Schale 10 niemals erreicht werden.

Um den Antrieb des Wascharms 3 trotz der in die gleiche Richtung gerichteten ersten Strahlen 5a, 5b der Wascharm-Hälften 3a, 3b sicher zu stellen, sind an den jeweiligen freien Enden der Wascharm-Hälften 3a, 3b horizontal gerichtete zweite Austritts-Düsen 61 derart angeordnet, dass hieraus austretende zweite Waschflüssigkeitsstrahlen 6 bei Betrachtung von einem Ende des Wascharmes 3 aus in beiden Hälften 3a, 3b in einander entgegengesetzte, im Wesentlichen horizontale Richtungen gerichtet sind und den Antrieb des Wascharmes 3 um die Drehachse der Drehkupplung 2 bewirken.

Bei der Ausführungsform nach den Figuren 3 und 4 weisen die Wascharme 3 einen im wesentlichen kreisrunden Querschnitt auf, und die ersten Strahlen 5a, 5b erzeugenden Düsen sind durch Austritts-Bohrungen 31 gebildet, die entlang einer Linie parallel zu einer Längsachse des Wascharmes 3 unter einem Radius ausgerichtet sind, der einen Winkel von mehr als 0 Grad und weniger als 90 Grad gegenüber einem Radius parallel zur Drehachse des Wascharms 3 aufweist. Dies ermöglicht eine besonders einfache Konstruktion unter Verwendung von Standardbauteilen.

Der Wascharm könnte jedoch in gleicher Weise einen anderen Querschnitt und/oder einzelne Düsen aufweisen, die in die Austrittsbohrungen 31 eingesetzt sind

Die freien Enden der Wascharm-Hälften 3a, 3b sind bei der Ausführungsform nach den Figuren 3 und 4 durch Endkappen 7 verschlossen, in denen zusatzlich zu den horizontal gerichteten zweiten Austrittsöffnungen oder Düsen 61 dritte Austrittsöffnungen oder Düsen 71 derart angeordnet sind, dass in Längsrichtung der Wascharme 3 gerichtete dritte Waschflüssigkeitsstrahlen erzeugt werden.

An den Endkappen 7 können weiterhin Halter 20 für zumindest einen Magneten 21 derart angeordnet sind, dass der zumindest eine Magnet 21 gegenüber der Längsachse der Wascharm-Hälften 3a, 3b achsversetzt angeordnet ist, wobei die Magnete mit ortsfest angeordneten Magnet-Sensoren für eine Überwachung der Drehung des Wascharms 3 zusammenwirken.

Obwohl in den Figuren 3 und 4 ein Wascharm 3 mit einer diesem zugeordneten Drehkupplung 2 gezeigt ist, kann dieser Wascharm 3 mit der beschriebenen Anordnung der ersten Austrittsöffnungen 31 auch Teil einer Wascharm-Anordnung sein, die mehrere an einem vertikalen Tragrohr angeordnete Wascharme 3 bzw. deren in entgegengesetzte Richtungen weisende Hälften 3a, 3b aufweist, die jeweils an dem Tragrohr befestigt sind und mit diesem in Strömungsmittelverbindung stehen, wobei die Drehkupplung an einem unteren und/oder oberen Ende des Tragrohres angeordnet ist.

Durch die gemeinsame Anordnung und Speisung der jeweiligen Wascharme in den einzelnen Etagen oder Ebenen eines Aufnahmewagens 1 mit Hilfe eines gemeinsamen Tragrohres wird eine wesentliche Vereinfachung der Konstruktion des Aufnahmewagens 1 erreicht, da nicht an jeder einzelnen Etage oder Ebene getrennte Lagerungen für Wascharme sowie Zuführeinrichtungen für das Strömungsmedium an diese Wascharme vorgesehen werden müssen.

Da lediglich eine obere und untere Lagerung des Tragrohres vorgesehen sein muss, ergibt sich weiterhin eine geringere Reibung der gesamten Wascharm-Anordnung, so dass sich diese Wascharme leichter bei einem vorgegebenen Strömungsmitteldruck drehen können. Die im Wesentlichen horizontal ausgerichteten Austrittsöffnungen 61 sämtlicher Wascharme tragen hierbei zum Antrieb der Wascharm-Anordnung bei.

## Patentansprüche

1. Wascharm für eine Reinigungsmaschine zum Reinigen von medizinischen, pharmazeutischen und/oder Labor-Utensilien, wobei der Wascharm (3) durch eine Drehkupplung (2) zur Dreh-Lagerung des Wascharms (3) um eine im Wesentlichen vertikale Drehachse und zur Zuführung von Waschmittel und/oder Trocknungsluft in zwei Hälften (3a, 3b) unterteilt ist, die jeweils erste Austritts-Düsen (31) zur Erzeugung von Waschflüssigkeits-Strahlen (5a, 5b 6) mit einer vorgegebenen Strahlrichtung aufweisen, die unter einem Winkel von mehr als 0 Grad und weniger als 90 Grad zur Drehachse der Drehkupplung verläuft, und wobei an den jeweiligen freien Enden der Wascharm-Hälften (3a, 3b) horizontal gerichtete zweite Austritts-Düsen (61) derart angeordnet sind, dass hieraus austretende zweite Waschflüssigkeitsstrahlen (6) bei Betrachtung von einem Ende des Wascharms (3) aus in beiden Hälften (3a, 3b) in einander entgegengesetzte, im Wesentlichen horizontale Richtungen gerichtet sind und den Antrieb des Wascharmes (3) um die Drehachse der Drehkupplung (2) bewirken, **dadurch gekennzeichnet, dass** die ersten Austritts-Düsen (31) in beiden Hälften (3a, 3b) des Wascharmes (3) zur Erzeugung von ersten Waschflüssigkeits-Strahlen entlang einer Linie parallel zu einer Längsachse des Wascharms derart ausgerichtet sind, dass die Strahlrichtungen bei Betrachtung von einem Ende des Wascharmes (3) aus in beiden Hälten (3a, 3b) in die gleiche Richtung und unter einem Winkel von mehr als 0 Grad und weniger als 90 Grad gegenüber einem zur Drehachse der Wascharme (3) parallelen Radius der Wascharme gerichtet sind.

2. Wascharm nach Anspruch 1, **dadurch gekennzeichnet, dass** die Wascharme (3) in einem Waschraum der Reinigungsmaschine und/oder an einem in den Waschraum einsetzbaren Aufnahmewagen für Waschgut drehbar angeordnet sind.

3. Wascharm nach Anspruch 1 oder 2, **dadurch gekennzeichnet, dass** die Wascharme (3) einen im wesentlichen kreisrunden Querschnitt aufweisen, und dass die ersten Düsen (31) durch Austritts-Bohrungen gebildet sind, die entlang einer Linie parallel zu einer Längsachse des Wascharmes (3) unter einem Radius ausgerichtet sind, der einen Winkel von mehr als 0 Grad und weniger als 90 Grad gegenüber einem Radius parallel zur Drehachse des Wascharms (3) aufweist.

4. Wascharm nach Anspruch 1, 2 oder 3, **dadurch gekennzeichnet, dass** die freien Enden der Wascharm-Hälften (3a, 3b) durch Endkappen (7) verschlossen sind.

5. Wascharm nach Anspruch 4, **dadurch gekennzeichnet, dass** in den Endkappen (7) dritte Austritts-Düsen (71) derart angeordnet sind, dass in Längsrichtung der Wascharme (3) gerichtete dritte Waschflüssigkeitsstrahlen erzeugt werden.

6. Wascharm nach Anspruch 4 oder 5, **dadurch gekennzeichnet, dass** an den Endkappen (7) Halter (20) für zumindest einen Magneten (21) derart angeordnet sind, dass der zumindest eine Magnet (21) gegenüber der Längsachse der Wascharm-Hälften (3a, 3b) achsversetzt angeordnet ist, und dass die Magnete mit ortsfest angeordneten Magnet-Sensoren für eine Überwachung der Drehung des Wascharms (3) zusammenwirken. ,

7. Wascharm nach einem der vorhergehenden Ansprüche, **dadurch gekennzeichnet, dass** der Wascharm (3) Teil einer Wascharm-Anordnung ist, die mehrere an einem vertikalen Tragrohr angeordnete Wascharme (3) aufweist, die jeweils mit dem Tragrohr in Strömungsmittelverbindung stehen, wobei die Drehkupplung an einem unteren und/oder oberen Ende des Tragrohres angeordnet ist.

## Claims

1. A wash arm for a cleaning machine for cleaning medical, pharmaceutical, and/or laboratory utensils, wherein the wash arm (3) is divided into two halves (3a, 3b) by a rotary coupling (2) for rotationally supporting the wash arm (3) around an essentially vertical rotational axis and for supplying washing agent and/or drying air, each half (3a, 3b) comprising first outlet nozzles (31) for producing jets of washing fluid (5a, 5b, 6) with a predetermined jet direction, which extends at an angle of more than 0° and less than 90° to the rotational axis of the rotary coupling, and wherein horizontally-oriented second outlet nozzles (61) are arranged at the free ends of the respective wash arm halves (3a, 3b) in such a way that, when looked at from one end of the wash arm (3), second spraying-liquid jets emerging essentially horizontally from the nozzles in the in the two half arms in opposite directions for causing the rotation of the wash arm (3) around the rotational axis of the rotary coupling (2), **characterised in that** the first outlet nozzles (31) in the two half arms (3a, 3b) of the wash arm (3) for producing first spraying-liquid jets are oriented along a line parallel to the longitudinal axis of the wash arm in such a way that, when looked at from one end of the wash arm (3), the jets in the two half-arms (3a, 3b) are all aimed in the same direction which is at an angle of more than 0 ° and less than 90° to a radius of the wash arms parallel to the rotational axis of the washing arms.

2. Wash arm according to claim 1, **characterised in that** the wash arms (3) are mounted rotatably in a washing chamber of the cleaning machine and/or on a trolley for the objects to be washed, adapted to be introduced into the washing chamber of the cleaning machine.

3. Wash arm according to claim 1 or 2, **characterised in that** the wash arms (3) comprise an essentially circular cross section, and **in that** the first nozzles (31) are formed by outlet holes, which are oriented along a line parallel to the longitudinal axis of the wash arm (3) on a radius at an angle of more than 0° and less than 90° to a radius parallel to the rotational axis of the wash arm (3).

4. Wash arm according to claims 1, 2, or 3, **characterised in that** the free ends of the wash arm-halves (3a, 3b) are sealed off by end caps (7).

5. Wash arm according to claim 4, **characterised in that** third outlet nozzles (7) are arranged in the end caps in such a way that third washing-liquid jets oriented in the longitudinal direction of the wash arms (3) are produced.

6. Wash arm according to claim 4 or 5, **characterised in that** holders (20) for at least one magnet (21) are mounted on the end caps (7) in such a way that the at least one magnet (21) is axially offset from the longitudinal axis of the washing arm-halves (3a, 3b), and **in that** the magnets cooperate with stationary magnetic sensors to monitor the rotation of the wash arm (3).

7. Wash arm according to any of the preceding claims, **characterised in that** the wash arm (3) is part of a washing arm assembly comprising several wash arms attached to a vertical support tube, the arms being in fluid-flow connection with the support tube, wherein the rotary coupling is arranged at the lower and/or upper end of the support tube.

## Revendications

1. Bras de lavage pour machine de nettoyage d'ustensiles médicaux, pharmaceutiques et/ou de laboratoire, le bras de lavage (3) étant divisé pour l'appui en rotation du bras de lavage (3) autour d'un axe de rotation sensiblement vertical et l'acheminement de produit nettoyant et/ou d'air de séchage par un accouplement rotatif (2) en deux moitiés (3a, 3b) qui présentent respectivement de premières buses de sortie (31) pour générer des jets de liquide de lavage (5a, 5b, 6) ayant un sens de projection prédéfini qui s'étend suivant un angle de plus de 0 degré et moins de 90 degrés par rapport à l'axe de rotation de l'accouplement rotatif et étant disposées aux extrémités respectivement libres des moitiés de bras de lavage (3a, 3b) de secondes buses de sortie (61) dirigées horizontalement de manière à ce que de seconds jets de liquide en sortant (6), observés depuis une extrémité du bras de lavage (3) dans les deux moitiés (3a, 3b), soient dirigés dans des sens opposés sensiblement horizontaux et provoquent l'entraînement du bras de lavage (3) autour de l'axe de rotation de l'accouplement rotatif (2), **caractérisé en ce que** les premières buses de sortie (31), dans les deux moitiés (3a, 2b) du bras de lavage (3), pour générer de premiers jets de liquide de lavage, soient orientées le long d'une ligne parallèle à un axe longitudinal du bras de lavage de manière à ce que les sens des jets, observés depuis une extrémité du bras de lavage (3), soient dirigés dans les deux moitiés (3a, 3b) dans le même sens et suivant un angle de plus de 0 degré et de moins de 90 degrés par rapport à un rayon des bras de lavage (3) parallèle à l'axe de rotation des bras de lavage.

2. Bras de lavage selon la revendication 1, **caractérisé en ce que** les bras de lavage (3) sont disposés de manière à pouvoir tourner dans un compartiment de lavage de la machine de nettoyage et/ou sur un chariot récepteur d'articles à laver pouvant être inséré dans le compartiment de lavage.

3. Bras de lavage selon la revendication 1 ou 2, **caractérisé en ce que** les bras de lavage (3) ont une section transversale sensiblement circulaire et que les premières buses (31) sont constituées par des trous de sortie qui sont orientés le long d'une ligne parallèle à un axe longitudinal du bras de lavage (3) suivant un rayon qui décrit un angle de plus de 0 degré et de moins de 90 degrés par rapport à un rayon parallèle à l'axe de rotation du bras de lavage (3).

4. Bras de lavage selon la revendication 1, 2 ou 3, **caractérisé en ce que** les extrémités libres des moitiés de bras de lavage (3a, 3b) sont fermées par des capuchons (7).

5. Bras de lavage selon la revendication 4, **caractérisé en ce que**, dans les capuchons (7), de troisièmes buses de sortie (7) sont disposées de manière à générer de troisièmes jets de liquide orientés dans le sens longitudinal des bras de lavage (3).

6. Bras de lavage selon la revendication 4 ou 5, **caractérisé en ce que**, au niveau des capuchons (7), des supports (20) pour au moins un aimant (21) sont disposés de manière à ce que l'au moins un aimant (21) soit disposé en décalage par rapport à l'axe relativement à l'axe longitudinal des moitiés de bras de lavage (3a, 3b) et que les aimants coopèrent avec des capteurs d'aimants disposés fixement pour surveiller la rotation du bras de lavage (3).

7. Bras de lavage selon une des revendications précédentes, **caractérisé en ce que** le bras de lavage (3) fait partie d'un dispositif de bras de lavage qui présente plusieurs bras de lavage (3) disposés sur un tube porteur vertical et qui sont respectivement en liaison de fluide d'écoulement avec le tuyau porteur, l'accouplement rotatif étant disposé à une extrémité inférieure et/ou supérieure du tuyau porteur.
